# EUROPEAN PATENT SPECIFICATION

(11) **EP 3 132 366 B1**
(45) Date of publication and mention of the grant of the patent: **12.07.2023**
(21) Application number: 15722769.5
(22) Date of filing: 02.04.2015
(51) Int. Cl.: G16H 10/60, G16H 50/20, G16Z 99/00

(54) **CONTROLLING ACTIONS PERFORMED ON DE-IDENTIFIED PATIENT DATA OF A CLOUD BASED CLINICAL DECISION SUPPORT SYSTEM (CDSS)**
STEUERUNG VON AKTIONEN, DIE AUF ANONYMISIERTEN PATIENTENDATEN EINES CLOUD-BASIERTEN KLINISCHEN ENTSCHEIDUNGSUNTERSTÜTZUNGSSYSTEMS (CDSS) DURCHGEFÜHRT WERDEN
COMMANDE D'ACTIONS RÉALISÉES SUR DES DONNÉES DE PATIENT ANONYMISÉES D'UN SYSTÈME DE SUPPORT DE DÉCISION CLINIQUE (CDSS) EN NUAGE

(30) Priority: 17.04.2014 US 201461980607 P
(43) Date of publication of application: 22.02.2017
(73) Proprietor: Koninklijke Philips N.V., 5656 AG Eindhoven (NL)
(72) Inventor: GROSS, Brian David, NL-5656 AE Eindhoven (NL); ELDO, Issac, NL-5656 AE Eindhoven (NL)
(74) Representative: Philips Intellectual Property & Standards
(86) International application number: PCT/IB2015/052422
(87) International publication number: WO 2015/159177

(56) References cited:
- WO-A2-2014/020490
- US-A1- 2011 153 351
- US-A1- 2011 283 110

## Description

The following generally relates to a clinical decision support system (CDSS) and more particularly to controlling actions performed on de-identified patient data located at a cloud based clinical decision support system (CDSS); however, the following can also be utilized with data other than de-identified patient data and/or with clinical and/or other non-clinical that is located at a location other than a cloud based clinical decision support system.

A clinical decision support system (CDSS) includes, for example, computer software that assists clinicians and/or other health professionals with decision making tasks such as determining a diagnosis of patient data. Cloud computing, generally, is the delivery of computing and/or storage capacity as a service to a community of end-recipients in which end users can access cloud-based applications through a web browser, a mobile application, etc., while the software and data are stored on servers at a remote location.

With a cloud based CDSS, computing on patient data and storage of at least some of the patient data is provided as a cloud service that assists clinicians and/or other health professionals with decision making tasks such as determining a diagnosis of patient data. Cloud providers manage the infrastructure and platforms on which applications run, and users utilize servers provided by cloud providers, system software to use in the servers, and/or application software and databases.

An approach for providing privacy and security of patient data has included using guaranteed unique identifications (GUIDS). With this approach, patient data stored at the cloud is de-identified in that patient identification (e.g., name, social security number, etc.) is replaced with a GUID. Access to the patient data and/or performing actions thereon is then controlled through the GUID. When de-identified data of interest is to be combined with non-de-identified data, the de-identified data is re-identified.

Unfortunately, known approaches may expose patient data to access by unauthorized users. As such, there is an unresolved need for approaches to controlling actions performed on de-identified patient data located at a CDSS, as well as data other than de-identified patient data and/or with clinical and/or other non-clinical that is located at a location other than a cloud based clinical decision support system.

US 2011/153351 A1 describes how a website may provide web services to a computer or device used by Record Producers, such as a hospital or doctors' office. The web services may be secured by "session ID tokens".

US 2011/283110 A1 describes a communication method between a client machine and a server. In described embodiments, the server generates a first security token, compares the first security token to a received second security token, and performs an action based on a result of the comparison.

Aspects described herein address the above-referenced problems and others.

The following describes an approach for providing privacy and security of patient data stored at a cloud based CDSS. In one instance, this includes using roles and tokens in which the roles define permissions for actions permitted on the patient data (e.g., view, run reports, re-identify, export, etc.) at a particular site, and the tokens allow the particular site to manage their user pool and prevent an unauthorized user from re-identifying de-identified patient data and to allow patient data to be re-identified based on user authentication by the "owning" site, without sending patient identification, ensuring the authenticated user request actually came from the "owning" site.

In one aspect, there is provided a method according to claim 1.

In another aspect, there is provided a method according to claim 9.

In another aspect, there is provided a system according to claim 16. The drawings are only for purposes of illustrating the preferred embodiments and are not to be construed as limiting the invention.
FIGURE 1 schematically illustrates an example system with a cloud-based CDSS and multiple sites, each site including a user authentication system and the cloud including a site authentication system.
FIGURE 2 schematically illustrates an example of the user authentication system.
FIGURE 3 illustrates an example method for site access to a cloud based CDSS from the perspective of the site.
FIGURE 4 illustrates an example method for site access to a cloud based CDSS from the perspective of the cloud based CDSS.

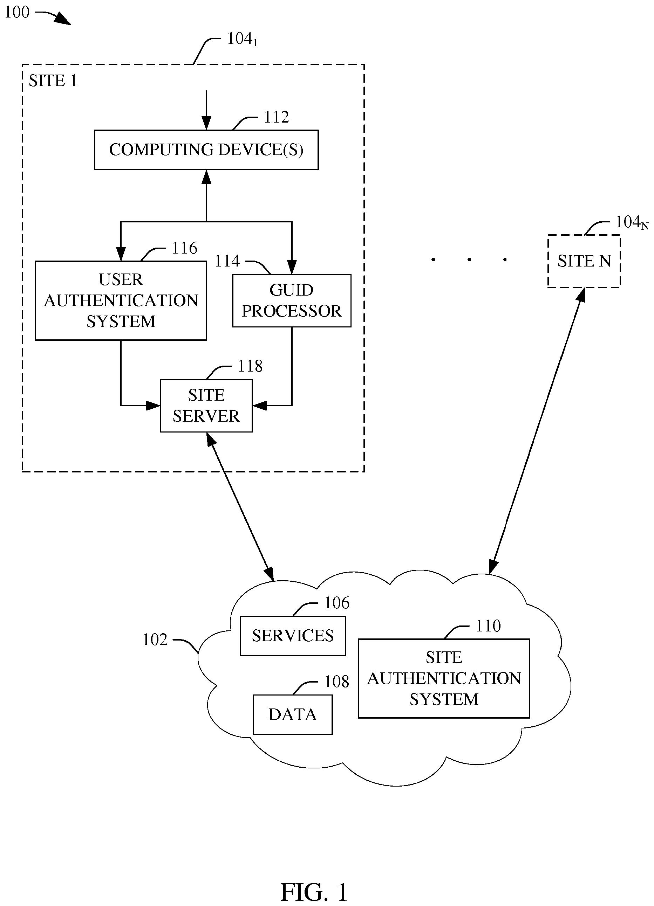

Initially referring to FIGURE 1, a system 100 includes a cloud-based CDSS 102 and N sites 104₁, .., 104_{N} (collectively referred to as sites 104), where N is an integer equal to or greater than one (1). As utilized herein, the term "site" refers to a facility that provides and/or accesses patient data and/or other information of the cloud-based CDSS 102. Examples of the sites 104 include, but are not limited to, a hospital, a clinic, a thru care, a doctors' office, an imaging center, etc.

The cloud-based CDSS 102 provides computing and/or storage services. This includes services 106, which are implemented by a microprocessor(s), a central processing unit(s), etc., and data 108, which is stored in a database or the like. An example of computing and/or storage services are described in application serial number PCT/IB2013/056055, filed July 24, 2013, and entitled "Federated patient guaranteed unique identification (guid) matching,".

The cloud-based CDSS 102 also includes a site authentication system 110. As described in greater detail below, the site authentication system 110 authenticates a site 104 prior to performing an action requested by the site 104 that requires site authentication. The site authentication system 110 is implemented via a processor (e.g., a microprocessor, a central processing unit, etc.) executing a computer executable instruction stored on computer readable storage medium, which excludes transitory medium.

A site 104 includes at least one computing device(s) 112. A computing device 112 includes one or more processors, computer readable storage medium such as physical memory, an input device (e.g., a mouse, a keyboard, etc.) and an output device (e.g., a display monitor). The computing device 112 resides in an admitting department, an emergency room, a laboratory, an intensive care unit, etc. An example of a computing device 112 includes a personal computer, a bedside monitor, a portable monitor, a hand-held monitor, a central monitoring station, etc.

A site 104 further includes a guaranteed unique identifier (GUID) processor 114, which generate a GUID for a patient. An example GUID includes an n-bit alphanumeric string that is based on a random number generated from a clock, etc. The GUID processor 114 also generates a mapping between GUIDs and patients. A GUID is used to replace patient identity to de-identify the patient data, for example, for privacy and security. An example of generating and using a GUID and a mapping is described in the application serial number PCT/IB2013/056055.

A site 104 further includes a user authentication system 116. As described in greater detail below, the user authentication system 116 facilitates authenticating a user of the computing device 112 and the site 104 for access to, manipulation of, retrieval of, re-identification of, etc. de-identified patient data of the cloud-based CDS system 102. The user authentication system 116 is implemented via a processor executing a computer executable instruction stored on computer readable storage medium.

A site 104 further includes a site server 118, which provides communication between the site 104 and the cloud-based CDS system 102. The server 118 de-identifies patient before conveying such data to the cloud-based CDS system 102. This includes replacing patient identity information with a GUID before conveying such data to the cloud-based CDSS 102. The de-identified patient data conveyed to the cloud-based CDSS 102 is non-identifiable in that it does not contain any information that identifies the patient's actual identity. In one instance, this mitigates privacy and/or security concerns.

The server 118 also re-identifies patient of the cloud-based CDS system 102. This includes replacing the GUID with the patient identity information. The re-identified patient data is identifiable in that it includes information that identifies the patient's actual identity. An example of de-identifying patient identity and associating a GUID with the de-identified patient data and re-identifying the patient identity is described in the application serial number PCT/IB2013/056055.

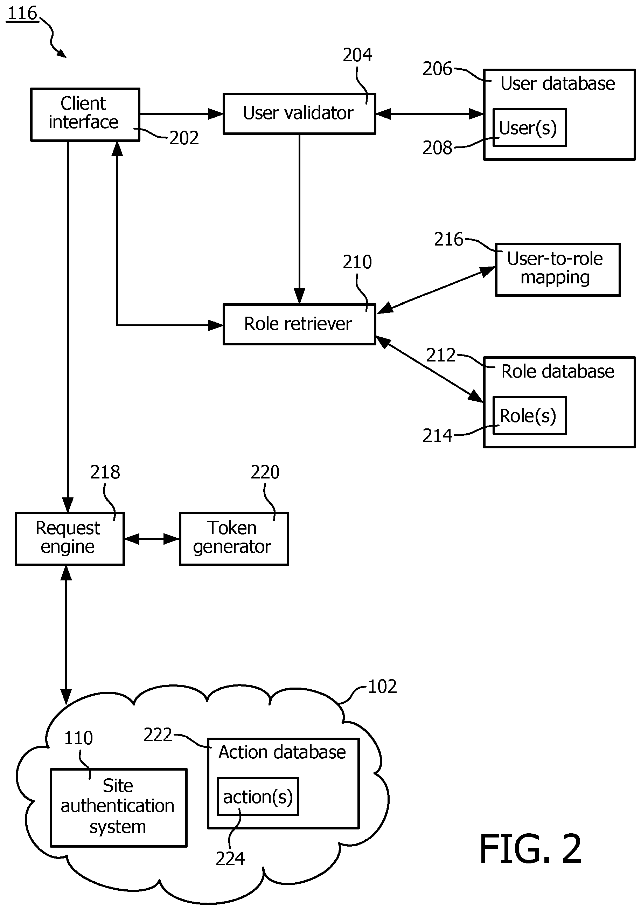

FIGURE 2 illustrates an example of the user authentication system 116 in connection with the site authentication system 110 of the cloud based CDSS 102.

The user authentication system 116 includes a client interface 202. The client interface 202 provides an interface between the computing device 112 and the user authentication system 116. The client interface 202 receives, from the computing device 112, cloud user log on information (e.g., a user name and password, bio-identification such as a fingerprint, electronic information from a magnetic or optical strip on an identification card, etc.) provided by a user to the computing device 112.

The user authentication system 116 further includes a user validator 204 and a user database 206 that stores a list of authorized users 208. The user validator 204 compares the cloud user log on information with the list of authorized users. In response to failing to match the cloud user log on information with an authorized user from the list of authorized users, the user validator 204 generates an invalidation signal or error message, which is conveyed to the client interface 202 and visually presented to the user via the computing device 112.

In response to matching the cloud user log on information with an authorized user from the list of authorized users, the user validator 204 generates a validation signal. The client interface 202, in response to receiving a validation signal, invokes retrieval of a role(s) assigned by the site 104 to the user. Examples of roles include: allow the user to see a list of types of reports; allow a user to view a report(s); allow a user to re-identify patient identity of a report; allow a user to export a report; allow a user to change a report, and/or other roles. Other and/or different roles are contemplated herein.

A role retriever 210 receives, from the client interface 202, a request for the role(s). A role database 212 stores a site defined role(s) 214, and a user-to-role mapping 216, which provides a mapping between cloud user log on information and a role(s). The roles allow the site 104 to control the actions performed on patient data. The role retriever 210 returns a list of the roles of the user to the client interface 202. The client interface 202 visually presents actions available to the user via the computing device 112.

A request engine 218 requests performance of an action by the cloud based CDSS 102 based on an invoked action from a list of available actions for the user. A token generator 220 generates a site session token for an action that requires site authentication. The site session token, for example, includes a unique identifier generated by the token generator 220 at the site 102 to identify an interaction session between the site 104 and the cloud based CDSS 102.

The site authentication system 110, in response to receiving a request, checks to see if the requested action requires site authentication. An action database 222 includes a list of actions 224 that require site authentication. If the action does not require site authentication, the cloud based CDSS 102 performs the action. If the action requires site authentication and does not include a session token, the cloud based CDSS 102 rejects the request and returns an error message.

In general, this allows the site 104 to manage the user pool, and permit who they want to have those action permissions without adding these users to the cloud authentication system. Furthermore, the session token prevents an unauthorized user or fraudulent user who may learns the user's log in information and roles in the cloud, from having the cloud based CDSS 102 perform an action such as re-identify de-identified patient data without first be authenticated by the site 104.

If the action requires site authentication and includes a session token, the site authentication system 110 sends a validation request, along with the session token, to the request engine 218. The request engine 218 compares the sent and the received session tokens and generates a signal indicating whether the sent and the received session tokens match (i.e., they are the same) or whether the sent and the received session tokens do not match (i.e., they are not the same).

The site authentication system 110, in response to receiving a session token validation signal, performs the action, invokes the cloud based CDSS 102 to perform the requested action. For example, where the request is to re-identify patient data in a particular report, the patient data is re-identified using the GUID. The site authentication system 110 can send the request engine 218 a notification indicating that a requested action has been performed by the cloud based CDSS 102.

Where a site 104 does not include the token generator 234, the site 104 will not have access to actions requiring site authentication. Furthermore, different sites 104 may have different roles and/or different actions requiring site authentication. Moreover, a user directly logging in to the cloud based CDSS 102, even where the user is authenticated, will not be able access actions requiring site authentication since the user is not requesting the action from a site 104.

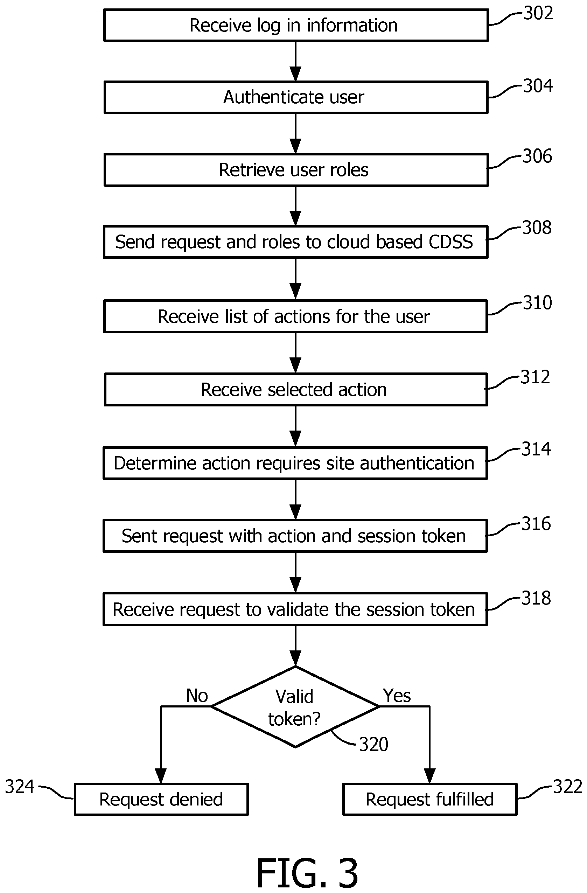

FIGURE 3 illustrates an example method for site access to a cloud based CDSS from the perspective of the site.

It is to be appreciated that the ordering of the acts in the methods described herein is not limiting. As such, other orderings are contemplated herein. In addition, one or more acts may be omitted and/or one or more additional acts may be included.

At 302, user log in information is provided, via a user of a computing device 112 at a site 104, to the user authentication system 116 at the site 104.

At 304, the user authentication system 116 authenticates the user based on the user log in information.

At 306, the user authentication system 116 retrieves roles assigned to the user based on the user log in information.

At 308, the user authentication system 116 sends a request, along with the roles, to the cloud based CDSS 102 for a list of actions available to the user.

At 310, the user authentication system 116 receives, from the cloud based CDSS 102, a list of actions available to the user, and visually presents the list via the computing device 112. As discussed herein, one or more of the actions in the list of actions may require site authentication. The received list of actions identifies which actions, if any, require site authentication.

At 312, the computing device 112 receives an input selecting one of the actions from the list.

At 314, the user authentication system 116 determines the selected action requires site authentication.

At 316, the user authentication system 116 sends, to the cloud based CDSS, a request that includes the action and a session token

At 318, the user authentication system 116 receives, from the cloud based CDSS 102, a request to validate the session token.

At 320, the user authentication system 116 validates the token if the sent and the received session tokens match (i.e., if they are the same).

At 322, if the user authentication system 116 validates the session token, the request is fulfilled by the cloud based CDSS 102.

At 324, if the user authentication system 116 does not validate the session token, the request is rejected by the cloud based CDSS 102.

The above method may be implemented by way of computer readable instructions, encoded or embedded on computer readable storage medium, which, when executed by a computer processor(s), cause the processor(s) to carry out the described acts. Additionally or alternatively, at least one of the computer readable instructions is carried by a signal, carrier wave or other transitory medium.

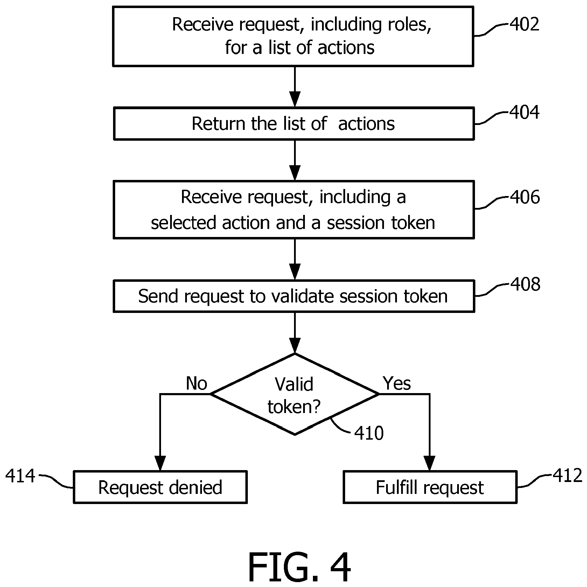

FIGURE 4 illustrates an example method for site access to a cloud based CDSS from the perspective of the cloud based CDSS.

It is to be appreciated that the ordering of the acts in the methods described herein is not limiting. As such, other orderings are contemplated herein. In addition, one or more acts may be omitted and/or one or more additional acts may be included.

At 402, the cloud based CDSS 102 receives a request, along with a list of roles for a user, from the user authentication system 116 at the site 104 for a list of available actions for an authenticated user.

At 404, the cloud based CDSS 102 returns the list of available actions.

At 406, the cloud based CDSS 102 receives a request for an action along with a session token.

At 408, the cloud based CDSS 102 sends a request, along with the session token, to the site 104 to valid the session token.

At 410, it is determined if the session token is validated.

At 412, if the session token is validated, for example, if the sent session token matches the received session token, the cloud based CDSS 102 fulfills the request.

At 414, if the session token is not validated, for example, if the sent session token does not match the received session token, the cloud based CDSS 102 rejects the request.

The above method may be implemented by way of computer readable instructions, encoded or embedded on computer readable storage medium, which, when executed by a computer processor(s), cause the processor(s) to carry out the described acts. Additionally or alternatively, at least one of the computer readable instructions is carried by a signal, carrier wave or other transitory medium.

The invention has been described with reference to the preferred embodiments. Modifications and alterations may occur to others upon reading and understanding the preceding detailed description. It is intended that the invention be constructed as including all such modifications and alterations insofar as they come within the scope of the appended claims.

## Claims

1. A method of requesting, by a site (104), an action on de-identified data to be performed by a cloud based clinical decision support system (102), the method comprising:
transmitting (316), by the site (104), a first request and a first session token, generated by the site (104), to the cloud based clinical decision support system (102), wherein the first request is for the action on de-identified data stored at the cloud based clinical decision support system, wherein the action is to be performed by the cloud based clinical decision support system (102), and wherein the action requires authentication of the site (104) by the cloud based clinical decision support system (102);
receiving (318), by the site (104), a second request and a second session token transmitted by the cloud based clinical decision support system (102) to the site (104), wherein the second request requests validation of the second session token;
comparing (320), by the site (104), the first and second session tokens, to generate, by the site (104), a validation signal indicating whether or not the first and second session tokens match;
transmitting, by the site (104), the validation signal to the cloud based clinical decision support system (102); and
only if the validation signal indicates that the first and second session tokens match, receiving (322), by the site (104), an indication that the cloud based clinical decision support system performed the requested action on said de-identified data stored at the cloud based clinical decision support system.

2. The method of claim 1, wherein the action includes a request to re-identify de-identified patient data.

3. The method of any of claims 1 to 2, wherein the action includes a request for one or more of: a list of possible actions; a list of actions available to the user; generation of a report; export of a report, or modification of a report.

4. The method of any of claims 1 to 3, further comprising:
receiving (302), at the site, cloud log in information;
authenticating (304) the cloud log in information at the site; and
transmitting the first request only in response to the cloud log in information being authenticated, wherein the cloud log in information at the site is authenticated prior to transmitting the first request.

5. The method of claim 4, further comprising:
retrieving (306), by the site, roles of an authenticated user.

6. The method of claim 5, further comprising:
discarding the first request only in response to the cloud log in information failing authentication.

7. The method of claim 6, further comprising:
transmitting (308), by the site (104), a third request to the cloud based clinical support system (102), wherein the third request includes the retrieved roles and includes a request for action available to the authenticated user, and the third request is received before the second request.

8. The method of claim 7, further comprising:
receiving (310), at the site (104), the requested action available to the authenticated user from the cloud based clinical support system (102).

9. A method of performing an action on de-identified data by a cloud based clinical decision support system (102), the method comprising:
receiving (406), by the cloud based clinical decision support system (102), a first request and a first session token transmitted by a site (104), wherein the first request is for the action on de-identified data stored at the cloud based clinical decision support system (102), wherein the action is to be performed by the cloud based clinical decision support system (102), and wherein the action requires authentication of the site (104) by the cloud based clinical decision support system (102);
transmitting (408), by the cloud based clinical decision support system (102), a second request and a second session token to the site (104), wherein the second request requests validation of the second session token;
receiving (410), by the cloud based clinical decision support system (102), a validation signal indicating whether or not the first and the second session tokens match;
performing (412), by the cloud based clinical decision support system (102), the requested action in response only to the validation signal indicating that the first and the second session tokens match; and
transmitting, by the cloud based clinical decision support system (102), a signal to the site (104) indicating the action has been performed by the cloud based clinical decision support system (102).

10. The method of claim 9, wherein the action includes a request to re-identify de-identified patient data.

11. The method of any of claims 9 to 10, wherein the action includes a request for one or more of: a list of possible actions; a list of actions available to the user; generation of a report; export of a report, or modification of a report.

12. The method of any of claims 9 to 11, further comprising:
receiving, at the cloud based clinical decision support system, a second request from the site, wherein the second request is received before the first request and includes roles assigned to an authenticated user and a request for actions available to the authenticated user.

13. The method of claim 12, further comprising:
transmitting, by the cloud based clinical decision support system, the requested action available to the site.

14. The method of any of claims 9 to 13, further comprising:
rejecting the first request in response only to the first and the second session tokens not being the same session token.

15. The method of claim 14, further comprising:
transmitting, by the cloud based clinical decision support system, an error message to the site, wherein the error message indicates the request has not been fulfilled because the first and second session tokens did not match.

16. A system (102), comprising:
a site (104) adapted to perform the method of any of claims 1 to 9, including:
a computing device (112); and
a user authentication system (116); and
a cloud based clinical decision support system (102) adapted to perform the method of any of claims 10 to 16, including:
a site authentication system (110),
wherein the user authentication system and the site authentication system control access by the site to de-identified patient data stored at the cloud based clinical decision support system.

17. The system of claim 16, wherein the action includes a request for one or more of: re-identification of de-identified patient data; a list of possible actions; a list of actions available to the user; generation of a report; export of a report, or modification of a report.

## Patentansprüche

1. Ein Verfahren, um durch eine Website (104) eine Maßnahme bezüglich anonymisierter Daten anzufordern, die durch ein cloudbasiertes klinisches Entscheidungsunterstützungssystem (102) durchgeführt werden sollen, das Verfahren umfasst:
Übertragen (316) einer ersten Anfrage und eines ersten Sitzungstokens durch die Site (104), von der Site erzeugt (104), an das cloudbasierte klinische Entscheidungsunterstützungssystem (102), wobei die erste Anfrage die Maßnahme für anonymisierte Daten betrifft, die im cloudbasierten klinischen Entscheidungsunterstützungssystem gespeichert sind, bei dem die Maßnahme vom cloudbasierten klinischen Entscheidungsunterstützungssystem (102) ausgeführt werden soll, und wobei die Maßnahme eine Authentifizierung der Website (104) durch das cloudbasierte klinische Entscheidungsunterstützungssystem (102) erfordert;
Empfangen (318) an die Website (104) einer zweiten Anfrage und eines zweiten Sitzungstokens, die durch das cloudbasierte klinische Entscheidungsunterstützungssystem (102) an die Website (104) übertragen werden sollen, wobei die zweite Anfrage eine Validierung des zweiten Sitzungstokens anfordert;
Vergleichen (320) der ersten und zweiten Sitzungstokens durch die Site (104), um durch die Site (104) ein Validierungssignal zu erzeugen, das angibt, ob das erste und das zweite Sitzungstoken übereinstimmen; Übertragen des Validierungssignals durch die Website (104) an das cloudbasierte klinische Entscheidungsunterstützungssystem (102); und nur wenn das Validierungssignal angibt, dass die ersten und zweiten Sitzungstoken übereinstimmen, Empfang (322), von der Site (104), eines Hinweises darauf, dass das cloudbasierte klinische Entscheidungs-Unterstützungssystem die angeforderte Maßnahme mit den besagten in der Cloud gespeicherten anonymisierten Daten im cloudbasierten klinischen Entscheidungsunterstützungssystem durchgeführt hat.

2. Verfahren nach Anspruch 1, wobei die Maßnahme eine Aufforderung zur erneuten Identifizierung der anonymisierten Patientendaten umfasst.

3. Verfahren nach einem der Ansprüche 1 bis 2, wobei die Maßnahme eine Anfrage umfasst nach einer oder mehreren von: Einer Liste möglicher Maßnahmen; eine Liste der Maßnahmen, die dem Benutzer zur Verfügung stehen; Erzeugen eines Berichts; Export eines Berichts oder Änderung eines Berichts.

4. Das Verfahren nach Anspruch 1 bis 3, ferner umfassend:
Empfangen (302) der Cloud-Anmeldeinformationen an der Website; Authentifizieren (304) der Cloud-Anmeldeinformationen an der Website; und
Senden der ersten Anfrage nur als Reaktion darauf, dass die Cloud-Anmeldeinformationen authentifiziert werden, wobei die Cloud-Anmeldeinformationen an der Website vor Übermittlung der ersten Anfrage authentifiziert werden.

5. Das Verfahren nach Anspruch 4, ferner umfassend:
Abrufen (306) der Rollen eines authentifizierten Benutzers durch die Site.

6. Das Verfahren nach Anspruch 5, ferner umfassend:
Verwerfen der ersten Anfrage nur als Reaktion darauf, dass die Authentifizierung der Cloud-Anmeldeinformationen fehlgeschlagen ist.

7. Das Verfahren nach Anspruch 6, ferner umfassend:
Übertragen (308) einer dritten Anfrage durch die Website (104) an das cloudbasierte klinische Entscheidungs-Unterstützungssystem (102), wobei die dritte Anfrage die abgerufenen Rollen umfasst und eine Anfrage nach Maßnahmen enthält, die dem authentifizierten Benutzer zur Verfügung stehen, und die dritte Anfrage wird vor der zweiten Anfrage empfangen.

8. Das Verfahren nach Anspruch 7, ferner umfassend: Empfangen (310) an der Website (104), der angeforderten Maßnahme, die dem authentifizierten Benutzer zur Verfügung steht, aus dem cloudbasierten klinischen Entscheidungs-Unterstützungssystem (102).

9. Ein Verfahren zum Ausführen einer Maßnahme an anonymisierten Daten durch ein cloudbasiertes klinisches Entscheidungsunterstützungssystem (102), wobei das Verfahren umfasst:
Empfangen (406) durch das cloudbasierte klinische Entscheidungsunterstützungssystem (102), einer ersten Anfrage und eines ersten Sitzungstokens, das von einer Site (104) übertragen wird, wobei die erste Anfrage
nach der Maßnahme mit anonymisierten Daten ist, die im cloudbasierten klinischen Entscheidungsunterstützungssystem gespeichert sind (102), wobei die Maßnahme durch das cloudbasierte klinische Entscheidungsunterstützung (102) durchgeführt werden soll, und wobei die Maßnahme eine Authentifizierung der Site (104) durch das cloudbasierte klinische Entscheidungsunterstützungssystem (102) erfordert;
Übertragen (408) durch das cloudbasierte klinische Entscheidungsunterstützungssystem (102), einer zweiten Anfrage und eines zweiten Sitzungstokens an die Site (104), wobei die zweite Anfrage die Validierung des zweiten Sitzungstokens anfordert;
Empfangen (410) durch das cloudbasierte klinische Entscheidungsunterstützungssystem (102), eines Validierungssignals, das angibt, ob die ersten und zweiten Sitzungstoken übereinstimmen oder nicht;
Durchführung (412), durch das cloudbasierte klinische Entscheidungsunterstützungssystem (102), der angeforderten Maßnahme nur als Reaktion darauf, dass das Validierungssignal angibt, dass das erste und das zweite Sitzungstoken übereinstimmen; und
Übertragen, durch das cloudbasierte klinische Entscheidungsunterstützungssystem (102), eines Signals an die Website (104), das angibt, dass die Maßnahme vom cloudbasierten klinischen Entscheidungsunterstützungssystem (102) durchgeführt wurde.

10. Verfahren nach Anspruch 9, wobei die Maßnahme eine Aufforderung zur erneuten Identifizierung der anonymisierten Patientendaten umfasst.

11. Verfahren nach einem der Ansprüche 9 bis 10, wobei die Maßnahme eine Anfrage umfasst nach einer oder mehreren von: Einer Liste möglicher Maßnahmen; eine Liste der Maßnahmen, die dem Benutzer zur Verfügung stehen; Erzeugen eines Berichts; Export eines Berichts oder Änderung eines Berichts.

12. Das Verfahren nach Anspruch 9 bis 11, ferner umfassend: Empfangen, im cloudbasierten klinischen Unterstützungsverfahren, einer zweiten Anfrage von der Site, wobei die zweite Anfrage vor der ersten Anfrage empfangen wird, und Rollen enthält, die einem authentifizierten Benutzer zugewiesen sind, und einer Anforderung für Maßnahmen, die dem authentifizierten Benutzer zur Verfügung stehen.

13. Das Verfahren nach Anspruch 12, ferner umfassend: Übertragen, durch das cloudbasierte klinische Entscheidungsunterstützungssystem, der angeforderten Maßnahme, die für die Site verfügbar ist.

14. Das Verfahren nach Anspruch 9 bis 13, ferner umfassend: Ablehnen der ersten Anfrage nur als Antwort darauf, dass die ersten und die zweiten Sitzungstoken nicht dieselben Sitzungstoken sind.

15. Das Verfahren nach Anspruch 14, ferner umfassend: Übertragen, durch das cloudbasierte klinische Entscheidungsunterstützungssystem, einer Fehlernachricht an die Site, wobei die Fehlermeldung darauf hinweist, dass die Anfrage nicht erfüllt wurde, weil der erste und der zweite Sitzungstoken nicht übereinstimmten.

16. System (102), umfassend: Eine Site (104), die dazu geeignet ist, das Verfahren nach einem der Ansprüche 1 bis 9 durchzuführen, einschließlich: Ein Computergerät (112); und ein Benutzerauthentifizierungssystem (116); und ein cloudbasiertes klinisches Entscheidungsunterstützungssystem (102), angepasst für die Durchführung des Verfahrens gemäß Anspruch 10 bis 16, einschließlich: Einem Benutzerauthentifizierungssystem (110), wobei das Benutzerauthentifizierungssystem und das Site-Authentifizierungssystem den Zugriff der Site auf anonymisierte Patientendaten enthält, die im cloudbasierten klinischen Entscheidungsunterstützungssystem gespeichert sind.

17. System nach Anspruch 16, wobei die Maßnahme eine Anforderung für eines oder mehrere umfasst von: Neuidentifizierung anonymisierter Patientendaten; eine Liste möglicher Maßnahmen; eine Liste von Maßnahmen, die die dem Benutzer zur Verfügung stehen; Erzeugen eines Berichts; Export eines Berichts oder Änderung eines Berichts.

## Revendications

1. Une méthode de requête, par un site (104), d'une action de dépersonnaliser les données devant être exécutées par le cloud d'un système d'aide à la décision clinique (102), la méthode comprend: la transmission (316), par le site (104), d'une première requête et un jeton de première séance, généré par le site (104), au système d'aide à la décision clinique (102) fondé sur le cloud, où la première requête est pour l'action sur des données stockées dépersonnalisées sur un système d'aide à la décision clinique fondé sur le cloud, où l'action doit être exécutée par un système d'aide à la décision clinique fondé sur le cloud (102), et où l'action requiert l'authentification du site (104) par le système d'aide à la décision clinique fondé sur le cloud (102); la réception (318), par le site (104), d'une seconde requête et d'un deuxième jeton de session transmis par le système d'aide à la décision clinique (102) d'un site fondé sur le cloud (104), où la seconde requête requiert la validation du jeton de deuxième session; la comparaison (320), par le site (104), d'un premier et second jeton de session, à générer, par le site (104), un signal de validation indiquant si le premier et second jeton de session correspondent ou non; la transmission, par le site (104), d'un signal de validation au système d'aide à la décision clinique fondé sur le cloud (102); et seulement si le signal de validation indique que le premier et second jeton de session correspondent, la réception (322), par le site (104), d'une indication que le système d'aide à la décision clinique fondé sur le cloud a exécuté l'action requise sur les données stockées dépersonnalisées dans le cloud d'un système d'aide à la décision clinique.

2. La méthode de la revendication 1, où l'action inclut une requête pour réidentifier des données de patient désidentifiées.

3. La méthode selon l'une quelconque des revendications 1 à 2, où l'action inclut une requête pour un ou plusieurs éléments suivants: une liste d'actions possibles; une liste d'actions disponibles à l'utilisateur: une production d'un rapport; l'exportation d'un rapport, ou la modification d'un rapport.

4. La méthode selon l'une quelconque des revendications 1 ou 3 comprend également: la réception (302), au site, d'informations de connexion au cloud; l'authentification (304) d'informations de connexion au cloud sur le site; et la transmission de la première requête seulement en réponse aux informations de connexion au cloud étant authentifiées, où les informations de connexion au cloud sur le site sont authentifiées avant de transmettre la première requête.

5. La méthode de la revendication 4 comprend également: la récupération (306), par le site, de rôles d'un utilisateur authentifié.

6. La méthode de la revendication 5 comprend également: le rejet de la première demande seulement en réponse aux informations de connexion où l'authentification échoue.

7. La méthode de la revendication 6 comprend également: la transmission (308), par le site (104), d'une troisième requête au système d'aide à la décision clinique fondé sur le cloud (102), où la troisième requête inclut les rôles récupérés et inclut une requête pour une action disponible auprès de l'utilisateur authentifié, et la troisième requête est reçue avant la seconde requête.

8. La méthode de la revendication 7 comprend également: la réception (310), sur le site (104), d'une action demandée disponible à l'utilisateur authentifié du système d'aide à la décision clinique fondé sur le cloud (102) .

9. Une méthode d'exécution d'une action sur les données dépersonnalisées par un système d'aide à la décision clinique fondé sur le cloud (102), la méthode comprend: la réception (406), par le système d'aide à la décision clinique fondé sur le cloud (102), une première requête et un premier jeton de session transmis par un site (104),où la première requête est pour une action sur les données stockées dépersonnalisées sur le système d'aide à la décision clinique fondé sur le cloud (102), où l'action doit être exécutée par le cloud fondé sur un système d'aide à la décision clinique fondé sur le cloud (102), et où l'action requiert l'authentification du site (104) du système d'aide à la décision clinique fondé sur le cloud (102); la transmission (408) par un système d'aide à la décision clinique basé sur le cloud (102) d'une seconde requête et un second jeton de session vers le site (104), où la seconde requête de validation requiert le deuxième jeton de session; la réception (410), d'un système d'aide à la décision clinique fondé sur le cloud (102), un signal de validation indiqué si les premiers et seconds jetons de session correspondent; l'exécution (412), par le système d'aide à la décision clinique fondé sur le cloud (102), l'action requise en réponse uniquement au signal de validation indiquant que le premier et second jeton de session correspondent; et transmettre, au système d'aide à la décision clinique fondé sur le cloud (102), un signal au site (104) indiquant que l'action a été exécutée par le système d'aide à la décision clinique fondé sur le cloud (102).

10. La méthode de la revendication 9, où l'action inclut une requête pour réidentifier des données de patient dé-identifiées.

11. La méthode comme revendiquée selon l'une quelconque des revendications 9 à 10, où l'action comprend une requête pour un ou plusieurs éléments suivants: une liste d'actions possibles; une liste d'actions disponibles à l'utilisateur; une production de rapport; l'exportation d'un rapport, ou la modification d'un rapport.

12. La méthode selon l'une quelconque des revendications 9 ou 11 comprend également: la réception, d'un système d'aide à la décision clinique fondé sur le cloud, une seconde requête du site, où la seconde requête est reçue avant la première requête et inclut des rôles assignés à un utilisateur authentifié et une requête pour des actions disponibles pour l'utilisateur authentifié.

13. La méthode de la revendication 12 comprend également: la transmission d'un système d'aide à la décision clinique fondé sur le cloud, l'action requise est disponible sur le site.

14. La méthode selon l'une quelconque des revendications 9 ou 13 comprend également: le rejet de la première requête en réponse uniquement aux premiers et seconds jetons de session n'étant pas les mêmes jetons de session.

15. La méthode de la revendication 14 comprend également: la transmission d'un système d'aide à la décision clinique fondé sur le cloud, d'un message d'erreur sur le site, où le message d'erreur indique que la requête n'a pas été satisfaite parce que les premiers et seconds jetons de session ne correspondent pas.

16. Un système (102) comprend: un site (104) adapté à exécuter la méthode selon l'une quelconque des revendications 1 à 9, comprenant: un dispositif informatique (112); et un système d'authentification de l'utilisateur (116); et un système d'aide à la décision clinique fondé sur le cloud (102) adapté pour exécuter la méthode selon l'une quelconque des revendications 10 à 16, comprenant: un système de site d'authentification (110), où le système d'authentification des utilisateurs et le système d'authentification du site contrôle l'accès par le site aux données dépersonnalisées des patients sont stockées sur le système d'aide à la décision clinique fondé sur le cloud.

17. Le système de la revendication 16, où l'action comprend une requête pour un ou plusieurs éléments: une réidentification des données dépersonnalisées des patients; une liste d'actions possibles; une liste d'actions disponibles pour l'utilisateur; la production d'un rapport; l'exportation d'un rapport; ou la modification d'un rapport.
